# EUROPEAN PATENT SPECIFICATION

(11) **EP 2 050 420 B1**
(45) Date of publication and mention of the grant of the patent: **19.09.2012**
(21) Application number: 08018028.4
(22) Date of filing: 15.10.2008
(51) Int. Cl.: A61F 5/453

(54) **Urinary device**
Harnvorrichtung
Dispositif urinaire

(30) Priority: 16.10.2007 GB 0720194
(43) Date of publication of application: 22.04.2009
(73) Proprietor: AIM-STRAIGHT LIMITED, HA1 3PA (GB)
(72) Inventor: Shelton, Michael, Harrow HA1 3PA (GB); Gunns, Graheme, Harrow HA1 3PA (GB); Maxwell, Peter, London SW1Y 6BL (GB)
(74) Representative: Ellis, Michael James

(56) References cited:
- WO-A-02/38088
- US-A- 2 878 486
- US-A1- 2003 195 483
- US-B1- 6 620 142

## Description

### FIELD OF THE INVENTION

The invention relates to a device for improving accuracy and hygiene during urination. The invention, in particular, relates to the provision of a urinary directional device for use by men when urinating standing up, which device has the effect of improving the hygiene of this procedure. The invention further relates to a method of manufacturing such a device.

### BACKGROUND OF THE INVENTION

In public and domestic toilet facilities, it is a common problem that spillage and misdirection during urination by an individual causes unpleasant and unhygienic stains and odours around the edges of the toilet or urinal, the surrounding floor area and even minor urine stains to the clothing of the individual. This is particularly a problem with children or the infirm or in toilets in public transport where the motion of the vehicle makes accuracy more difficult. A further problem is that where conventional toilet and hand washing facilities are not available, even if using a urinary directional device, there typically no opportunity to wipe up or hand wash which causes unpleasant staining or odour in clothing.

Several potential solutions to at least the former problem have been proposed, although no entirely satisfactory solution exists, some of which are described below.

JP 10-234763 discloses a male urination adjustment cylinder for correcting an orbit when males of advanced age urinate. The device described is cylindrical with a tapered distal end for discharge of urine. It is designed to be worn on the penis of the male urinating to improve directional flow and reduce soiling of the toilet bowl or clothing. The cylindrical portion includes a handle for the user to direct urination and to aid with application and removal. The described device, however, has the disadvantage that it has to be fitted substantially on to the penis which is a fiddly procedure and it does not address the hygiene issues of wiping or mopping excess urine remaining on the penis.

JP 2001-161734 describes an alternative solution to assist in directional urination for the male. Rather than the male wearing the device on his penis during urination, this document describes a guide cylinder of conical or other funnel-like configurations which is supported in a support structure positioned above the toilet bowl so that urination into the funnel will reduce scatter and avoid soiling of the toilet bowl or trousers. The guide cylinder portion may be disposable and formed of water dissolvable paper or may be reusable and formed of polyethylene or polypropylene to enable a lightweight element that is repeatably washable. The guide die typically has a length of from 10 to 50 cm, a large opening diameter of form 10 to 40 cm and a small opening diameter of 1 to 5 cm. The support structure portion consists of a movable arm attached at one end via a hinge to the under side of the toilet lid or to a wall via an extendable arm and having at the other end a retaining ring for supporting the cylinder guide portion. The support structure may be fitted with a non-slip material such as rubber, or a clamp, to reduce slippage of the guide cylinder portion in the support. There is no disclosure in JP 2001-161734 of the structure being movable such that the guide cylinder is in contact engagement with the penis and as such there is still the potential for the urine to spray or miss the funnel. The assembly also has additional hardware (e.g. the support structure or the funnel itself is reusable) which has the potential for gathering spilled urine and becoming a hygiene hazard itself. Furthermore, the document does not address the wiping of excess urine from the penis and surroundings nor does it provide for any anti-bacterial or anti-fungal function.

ES 2181547 describes a single use or disposable male urination device with a wall dispenser. The device is designed to prevent staining of the floor and surroundings and resulting bacteria and odour when men urinate standing up. The device, which is formed of a slightly waterproofed (e.g. by compression or waxing) cellulose or paper adapter, consists of a truncated cone which extends as a cylinder to form a one-piece Y-shaped form, or alternatively a simpler truncated V-shaped cone or a cylinder, which leads urine from the penis to the toilet. The described device is of length in the region of 50 cm, which in the case of a Y-shaped device is typically 25 cm of truncated cone and 25 cm cylinder portion. The large diameter opening, in which the penis engages, is typically about 5cm whilst the small diameter opening is typically about 1.5cm. A range of dispenser arrangements are described, including a stack of inter-stacking guide cylinders and a role of connected devices in a dispenser. A disadvantage of the device described in ES 2181547 is the length of the device may make it awkward to use and would increase the volume of residual urine in the device after use. There is no mention in this document as to the improvement of hygiene by wiping excess urine from the penis or any anti-bacterial function.

EP 1055402 discloses a female urinary device to allow women to urinate in an upright position. It comprises a foldable tubular body which in use assumes a reverse truncated pyramid form in which the apertures are preferably quadrangular or rhomboidal in shape. The purpose of the device is to enable females to urinate in a standing position, in particular to avoid contact with toilets in public conveniences which may not be hygienic. The device is preferably disposable, for hygiene reasons, but may be formed of material enabling reuse. The device is made of a material, e.g. paper or card, having a water impermeable or water repellant coating.

Several other disclosures, including GB 2361871, WO 2004/028322, WO 00/15166 and GB 2396819 describe urine directional devices, which may be disposable or reusable, typically designed to assist women in urinating standing up.

WO-A-02/38088 describes a disposable urinary directional device adapted to surround the body of the penis of the user to assist in directing urine in a desired manner (e.g. toward a lavatory bowl) to avoid spillage and associated staining and bad odours. The device may be of a truncated conical shape and typically comprises a deformable material in order that it may conform with the surface of the penis. The preferred embodiment described is a laminated structure having an outer layer of water-repellent material and an inner skin-contacting layer of urine absorbing material. In order that the user will automatically cleanse his hands whilst using the device, the device may be impregnated with an antiseptic agent (of the type used to impregnate hygienic wipes).

US-B-6620142 describes a urinary aid to help male users channel urine into a toilet. The aid comprises a conduit element for the passage of urine and an end section for receiving the penis and a tab portion for holding the aid in place. The end section for receiving the penis may comprise a coating to minimize wetting (e.g. by impregnation with a soap or fat or the like) and can be provide with an uncoated absorbent outer edge for wiping the penis after use.

Several prior art documents propose potential solutions to assist in reducing spraying and improving directional urination for men (and women) when urinating standing up. However, several of these potential solutions have disadvantages associated with them, such as the excessive length of the funnel. Furthermore, there is no adequate means to enable improved directional flow in men and thereby reduce spraying when urinating standing up whilst providing improved hygiene to the user.

### PROBLEM TO BE SOLVED BY THE INVENTION

There is therefore a problem of providing a urinary directional device that provides improved independence and hygiene for the user.

It is an object of this invention to provide such a device which enables the hands and urinogenital area of the user to remain hygienically clean or cleansed.

It is a further object of the present invention to provide a device that can be discarded at the site of use and reduce any bacterial build up or discomfort for the user.

### SUMMARY OF THE INVENTION

In accordance with a first aspect of the invention, there is provided a urinary directional device for improving the directional urination of a male user, said device comprising a conduit for the passage of urine and having a proximal end adapted for engagement with the penis of the user and having a proximal aperture to enable the passage of urine into the conduit and a distal end having a distal aperture to enable the passage of urine from the conduit, said device having an interior surface and an exterior surface, the device being characterized in that the interior surface provides one or more of an anti-bacterial or antiseptic or an anti-fungal function whereby the penis of the user can be cleansed by wiping on the interior surface of the device after urination.

In a second aspect of the invention, there is provided a method of manufacturing a urinary direction device described above , said method comprising providing in sheet form a material with which to form the device, impregnating or coating at least a portion of said material with an anti-bacterial or antiseptic or anti-fungal agent, cutting said sheet material and assembling therefrom the device described above.

In a third aspect of the invention, there is provided a stacking assembly of the devices described above, said assembly comprising a dispensing component for dispensing said devices and a plurality of said stacked devices.

### ADVANTAGES OF THE INVENTION

The present invention enables improved directional control of urination by men when urinating standing up, whilst also improving hygiene by enabling the user's penis to be cleansed by a portion of the device having an anti-bacterial/antiseptic and/or anti-fungal function and optionally a further cleansing function. By having the cleansing, anti-bacterial and/or anti-fungal function located at specific portions of the device, the cleansing action can be performed on the user simply by the action of removing the device after use. The device reduces spillage and soiling of the area surrounding a toilet or urinal and of the user's clothing as well as improving the personal hygiene of the user.

### BRIEF DESCRIPTION OF THE DRAWINGS

Fig. 1 is an aspect view of a directional urinary device of the present invention;
Fig. 2 is a plan view of the directional urinary device depicted in Figure 1;
Fig. 3 is a side view of the directional urinary device depicted in Figure 1;
Fig. 4 is a cross sectional view from A-B of the directional urinary device depicted in Figure 3.

### DETAILED DESCRIPTION OF THE INVENTION

The device according to the present invention may be used in any location as and when required. It is particularly useful for directional urination into a urinal or toilet bowl in domestic or public conveniences, where cleanliness and hygiene issues result from directional errors during urination. It also provides the additional benefit of anti-bacterial, antiseptic or anti-fungal function for the user.

In general, the invention provides a urinary directional device, for improving the directional urination of the user, or an insert for such a device, which is adapted for engagement with the urinogenital area of the user, which device or insert has on at least a portion thereof an anti-bacterial/antiseptic/anti-fungal and/or absorbent function whereby the user can wipe excess urine from the urinogenital area on removal following urination.

For further describing the invention and its various embodiments hereinafter, the terms proximal and distal shall be used to refer to positions on the device or insert relative to the user during use of the device.

The urinary directional device comprises a conduit for the passage of urine, has a proximal end adapted for engagement with the urinogenital area of the user and a proximal aperture to enable the passage of urine into the conduit and a distal end having a distal aperture to enable the passage of urine from the conduit. The device may further be defined as having a distal portion characterized by providing for the passage of a stream of urine and a proximal portion, which during urination does not come into substantial contact with the stream of urine. The device has an interior surface and an exterior surface. By interior surface, it is meant the surface defining an internal volume formed in the device by covering the distal and proximal apertures with a planar surface.

In a device according to the invention, the internal surface provides one or more of an anti-bacterial or antiseptic or an anti-fungal function. This is preferably achieved by coating and/or impregnating the interior surface with a formulation or composition having said function. Preferably, at least part of the proximal portion provides one or more of a cleansing, an anti-bacterial or antiseptic or an anti-fungal function whereby the user's penis can be cleansed by wiping with the at least part of the proximal portion after urination.

The invention may provide an insert into a reusable urinary directional device having a conduit for the passage of urine, a proximal end adapted for engagement with the urinogenital area of the user, a proximal aperture to enable the passage of urine into the conduit, a distal end having a distal aperture for the passage of urine from the conduit and having a distal portion characterized by providing for the passage of a stream of urine and a proximal portion, which during urination does not come into direct contact with the stream of urine, the insert is adapted to removably fit about and/or inside the proximal end of the device and provides over at least a part of the interior surface of the insert, preferably at least part of the proximal portion of the device provides a cleansing, an anti-bacterial/antiseptic or an anti-fungal function whereby the user's penis can be cleansed by wiping with the at least part of the proximal portion after urination.

The directional device according to the invention may be disposable or may be a reusable device fitted with the described disposable insert. It is a preferred feature of the invention that at least the soiled portion of a urinary directional device is used once and then disposed of after soiling. Similarly, the anti-bacterial/antiseptic or anti-fungal or absorbency features of the invention are intended for a single use.

As can be seen from the above the urinary directional device may comprise an insert which insert may provide an anti-bacterial/antiseptic and/or anti-fungal, whereby the user's penis can be cleansed on removal of the device after urination.

In the description that follows, the text shall refer to the urinary directional device according to the first aspect of the invention. However, where the context allows, it is intended also to refer to an insert for such a urinary directional device.

In an embodiment of the invention, the urinary directional device (or the insert therefore) is adapted for use by a man when urinating to improve directional accuracy. The device finds particular benefit for use by uncircumcised males. According to this embodiment, the proximal end is of a shape and dimension to allow the tip of the penis to fit, preferably snugly, within the proximal aperture and engage with the device (preferably so as to form a loose seal about the circumference of the penis) to allow, during urination, passage of urine through the conduit of the device and out the distal aperture. The proximal aperture may therefore be of any suitable shape with sufficient overall dimension, such as a quadrangle, pentagon, hexagon, a fluted shape or preferably an elliptical or circular shape to provide a better engagement.

The overall shape of the device according to this embodiment may be any suitable shape. For example the device may be an inverted pyramid (the larger end of which pyramid is the proximal end of the device) of triangular, quadrangular, pentagonal, hexagonal or other form or alternatively a fluted arrangement to encourage passage of urine rapidly through the conduit or a conical form. The device may further comprise of a conical or inverted pyramidal shape with a cylindrical or further pyramidal distal extension. Preferably, the device is a truncated conical (or frusto-conical) form in which the proximal aperture is formed as the base of the cone and the distal aperture is defined by the truncated peak of the cone.

The cleansing and/or anti-bacterial/antiseptic and/or anti-fungal function may be provided in any suitable arrangement on the interior surface, preferably on the proximal portion of the device. Preferably, it is provided such that on removal of the device by the male user, that part of the proximal portion comes into direct contact with the external terminus of the urethra and surrounding area providing an absorbency and/or cleansing effect. In one embodiment in which the device is substantially frusto-conical in shape, the absorbent and/or anti-bacterial/antiseptic and/or anti-fungal function is provided in a part of the proximal portion of the device which extends largely or more preferably fully around the interior surface of the device (e.g. in a frusto-conical form) to enable the benefit to be effected irrespective of the rotational positioning of the device by the user. Preferably, the longitudinal dimension (i.e. in the direction of the axis of the conduit) of the coating is in the range of from 10 to 50 mm, more preferably 15 to 30 mm. In another embodiment the entire interior surface (or internal face) is provided with the cleansing, anti-bacterial/antiseptic and/or anti-fungal function, preferably by coating or impregnation with a formulation or composition having such property.

Optionally, the device may further comprise of an extended tab (extending externally from the aperture, defined by a rim to the aperture), which in use may be positioned to the underside of the penis and optionally may be absorbent and/or anti-bacterial/antiseptic whereby on removal of the device after use, the tip of the penis may be cleansed further. Such a tab has the further benefit of increasing the ease of removal of the device from a stack (where the devices are dispensed from the proximal end of the device) or reducing the risk of multiple dispensing (where the devices are dispensed from the distal end of the device). For simplicity of manufacturing, however, it is preferred that no such tab is provided.

Optionally, the cleansing and/or anti-bacterial/antiseptic and/or anti-fungal function may also be provided on the exterior surface of the device to enable the hands of the user to be cleansed or kept sanitary.

The device according to this embodiment is preferably formed at its proximal end of a rolled edge to reduce abrasion and improve comfort, particularly when the device is formed of a paper or paper-like material.

The dimensions of the device according to this embodiment may be any suitable according to the circumstances. Preferably however, the device is of a length in the range 50 to 150 mm, more preferably 60 to 100 mm and most preferably in the range 65 to 80 mm. Preferably, the proximal aperture is in the range 40 to 75mm, more preferably 50 to 65 mm and most preferably 55 to 60 mm. The distal aperture is preferably in the range of from 5 to 30 mm, more preferably 10 to 25 mm and most preferably from about 15 to about 20 mm.

An insert for a reusable device may preferably take the shape at the proximal end of the respective proximal aperture of the reusable device or a portion thereof. An insert may comprise of an external lip or sleeve portion around a portion of the proximal aperture whereby the insert and lip or sleeve thereof engage with the reusable device such that the sleeve or lip slips over the proximal end to cover a portion of the exterior of the reusable device. An insert may be shaped to mimic the whole of internal surface of the reusable device to which it may be fitted, but preferably shaped to only partially cover the internal surface of the reusable device and is preferably shaped so as to provide coverage over the proximal portion (or at least a part thereof) whereby upon removal of the device the urinogenital area can come into contact with the insert and the anti-bacterial or anti-fungal or absorbent portion thereof in order to cleanse the user. For example, an insert into a urinary directional device may form a sleeve over at least the rear portion (in use) of the proximal aperture rim and/or may be shaped to fit at least a rear portion of the proximal portion of the device and provide thereon an absorbent and/or anti-bacterial/antiseptic and/or anti-fungal function whereby on removal of the device by the user, the insert and its hygienic function may be caused to come into contact with or may be conveniently contacted with the urinary area to absorb any residual urine and/or apply a hygienic substance to the urinogenital area about the terminus of the urinary tract. In this way, the insert can substantially improve the hygienic use of reusable urinary directional devices.

The insert may be shaped or adapted to fit to existing or new urinary directional devices (including, for example, a simple reusable truncated conical device) in order to improve the hygienic performance of the device.

The device according to the invention is preferably for a single use and as such is made of a disposable material, such as paper, cellulose or other fibrous material or of a plastic material. Preferably, the device is formed from a paper or cellulose material and, for environmental reasons, most preferably paper or other biodegradable material such that it can be flushed away or easily disposed of. In order to maintain robustness and integrity of the device during use, where the device is formed of a core material that can absorb liquid or is biodegradable (such as paper), it is preferable that at least the internal face of the distal portion and preferably also the proximal portion or the whole device, optionally other than an absorbent part of the proximal portion, is made water proof or water repellent, by, for example, compression, coating with a water proof or water repellent material or impregnating with a water proof or water repellent material. Preferably, the necessary degree of water-proofing (in order to assist in a single urination event) is achieved by compression of the paper material forming the device. Accordingly, the device, and in particular, the distal portion of the device repels water allowing urine to pass unimpeded through the device while the device retains its integrity until after use. Preferably, the proximal aperture is defined by a rim which has a rolled edge (especially if the device is formed of a paper or like product) to reduce abrasion and increase the comfort of the device in use.

In one embodiment, the at least part of the proximal portion of the device provides an absorbent and an anti-bacterial/antiseptic and/or anti-fungal function, especially an anti-bacterial or antiseptic function, which function should be hypoallergenic. The absorbent portion may be provided in the proximal portion of the device, for example where the device is formed of a an absorbent core material, by water proofing the device other than in part of the proximal portion which is to remain absorbent. Alternatively, the absorbent portion may be formed by laying on at least part of the internal surface of the proximal portion a layer or coating of an absorbent material, such as an absorbent hydrophilic material, textile or hydrophilic foam, or by coating on at least a portion of the internal surface a porous material such as a coating of silica or clay containing a hydrophilic mordant. Suitable layer or coating materials for use as the absorbent function, include wicking materials such as rayon acetate needled felting, single component fibres (or blended component fibre materials) comprising wool, cotton, rayon, nylon and/or polyester. Other suitable absorbent materials include foamed polymer materials such as polyurethane foams, meshes of synthetic polymers and flexible solids such as latex, but preferably the polymeric foam absorbents are hydrophilic in nature. Swellable polymers, such as polyvinyl alcohol, may also be used to provide the absorbent function. The absorbent function may alternatively be provided by a layer or coating (or impregnation) of an inorganic particulate material which provides a porous material, such as silica, clay, calcium sulfate or calcium carbonate or other such inorganic water absorbing material. Optionally, the absorbent material (such as the inorganic particulate, polymer foam or swellable polymer or even wickable fibre) may comprise or be impregnated with a mordant material capable of trapping the urine (especially the urea and ammonium compounds) thereby improving the effect of the absorbent material and reducing the odour associated with discarded used devices. As a further option, the absorbent layer or coating material may be further impregnated or imbibed with a fragrance, which is optionally released on contact with a liquid, to reduce problems with odour from used devices or to further provide a fragrance release effect for the public or domestic toilet. Where the absorbent function is provided by a layer/layers or a coating of absorbent or wickable material, it is preferably provided in a layer of up to 2.5 mm depth/thickness and more preferably from about 0.5 to about 2 mm.

Optionally, an absorbent coating or portion may be provided on the exterior surface of the device whereby any soiling of the users hand or fingers can be cleansed simply by use of the material.

In a preferred embodiment of the present invention, the device is capable of carrying advertisements primarily on the exterior surface. Typically, this will be achieved by ink-jet printing and as such the device is preferably manufactured from a medium having one surface (which will form the exterior surface of the device) capable of receiving an ink-jet image. Preferably, in order to provide a high quality image, the media from which the device is formed comprises on one surface an ink-jet receiving medium, which optionally may be a porous or a non-porous (i.e. swellable) medium.

Preferably, the media is a porous medium. By utilizing a porous medium, an image may be formed by a high speed ink-jet printing method whilst providing an additional function for the resultant device that the exterior surface of the device is highly absorbent to liquid (especially aqueous fluid) so that any splash or drip of water or urine is rapidly absorbed and not transferred to the user's hands and similarly that any water or urine on the users hands is rapidly absorbed.

Any suitable porous media may be used. Preferably, the porous medium is biodegradable or suitable for being flushed or otherwised disposed of.

The porous medium is typically formed of particulate material, typically inorganic particulate but optionally organic particles, bound by a resin. Any suitable resin may be used, such as polyvinyl alcohol. Optionally, the porous medium is a swellable polymer material having a network of pores formed therein (e.g. by coating the swellable polymer in the presence of a blowing agent which is then activated before curing). In this option, the swellable polymer is preferably a polyvinyl alcohol or other aqueous soluble polymer material.

Suitable such inorganic particulate materials may include, for example, one or more of silica (e.g. colloidal silica), alumina (e.g. alumina sols, colloidal alumina, cationic aluminium oxide or hydrates thereof, pseudoboehmite, etc.), surface-treated cationic colloidal silica, magnesium silicate, aluminium silicate, magnesium carbonate, kaolin, talc, calcium sulfate, barium sulfate, titanium dioxide, zinc oxide, zinc sulfide, zinc carbonate, satin white, diatomaceous earth, calcium silicate, aluminium hydroxide, lithopone, zeolites (such as molecular sieves 3A, 4A, 5A and 13X), hydrated hallocyte and magnesium hydroxide.

The binder may be any suitable binder capable of effectively binding the inorganic particular materials to form a porous ink-receiving layer capable of retaining a pigment or dye, preferably a pigment, to form a printed image having good image properties. Suitable such binders include, for example, one or more of naturally occurring hydrophilic colloids and gums such as gelatin, albumin, guar, xantham, acacia and chitosan and their derivatives, functionalised proteins, functionalised gums and starches, cellulose ethers and their derivatives, such as hydroxyethyl cellulose, hydroxypropyl cellulose and carboxymethyl cellulose, polyvinyl oxazoline and polyvinyl methyloxazoline, polyoxides, polyethers, poly(ethylene imine), poly(acrylic acid), poly(methacrylic acid), n-vinyl amides including polyacrylamide and polyvinyl pyrrolidone, polyethylene oxide and polyvinyl alcohol, its derivatives and copolymers and most preferably polyvinyl alcohol. Preferably, the binder is present in an amount as a ratio of inorganic particulate materials to binder of from 70:30 to 99:1, preferably 75:25 to 96:4 and still more preferably 85:15 to 95:5.

Other components which may be present in the ink-jet receiving medium include, for example, a surfactant and a mordant. Suitable mordants, which may be useful to bind the dye or pigment in the ink in the ink-receiving layer in order to improve still further the image density, include, for example, a cationic polymer, e.g. a polymeric quaternary ammonium compound, or a basic polymer, such as poly(dimethylaminoethyl)methacrylate, polyalkylenepolyamines, and products of the condensation thereof with dicyanodiamide, amine-epichlorohydrin polycondensates, divalent Group 11 metal ions, lecithin and phospholipid compounds or any suitable mordant that is capable of assisting with fixing a dye material transferred to it. Examples of such mordants include vinylbenzyl trimethyl ammonium chloride/ethylene glycol dimethacrylate, poly(diallyl dimethyl ammonium chloride), poly(2-N,N,N-trimethylammonium)ethyl methacrylate methosulfate, poly(3-N,N,N-trimethylammonium)propyl chloride. A preferred mordant would be a quaternary ammonium compound.

Optionally, the medium from which the device is made may comprise, on the side on which an image will be visible, an amorphous hydrated aluminosilicate, such as an allophane, for the reduction of smearing of an image when a printed receiver is stored at high temperatures and humidities, as may typically be the case for the present device which is likely to be used in washrooms.

In the embodiment of the invention, where the media forming the device comprises on one side (the exterior surface) an image-receiving capability, this is preferably achieved by providing a porous ink-receiving layer (as described above) on a suitable support. The non-image receiving surface of the support will typically define the interior surface of the device. Any suitable support in the circumstances may be used, such as a resin-coated support (e.g. resin coated paper), but is preferably a non resin-coated support, more preferably a non resin-coated paper.

In a preferred embodiment, the urinary directional device of the present invention has an image provided on the exterior surface. In a particularly preferable embodiment, due to the moist environment in wash rooms where the device will be used, the image formed is water fast to prevent blurring or smudging of the ink forming the image. A water-fast image may be achieved by any suitable means, but one such method is to utilize an epoxy resin in the image receiving surface of the media from which the device is formed and providing an epoxy curing agent with the ink during printing.

According to this preferred embodiment, in which the device according to the invention carries an image on the exterior surface, which may be a personalized message or advertising, it is preferred that the image is fixed to prevent blurring in a humid atmosphere as may be present in a washroom environment. The exterior surface carrying the image is preferably formed of a porous ink-receiving layer as defined above whereby the image is formed in good colour and resolution characteristics. The interior surface of the device may be compressed paper as discussed above or coated to be at least temporarily water resistant. The interior surface is provided with a cleansing, anti-bacterial/antiseptic and/or anti-fungal function, preferably by coating or impregnating the medium with a composition having the function, most preferably a powdered formulation (increasing the shelf life of the device as compared with liquid formulations which may dry out during storage).

The image may be provided on the exterior surface in any orientation desired. In one embodiment, however, it is preferred that the image is formed on the exterior surface such that it is viewed in the desired manner when the device is orientated distal end upward, such that when stacked distal end upward, it may be viewed at a beneficial angle.

An anti-bacterial or antiseptic or an anti-fungal function is preferably provided in at least part of the proximal portion of the device, on the interior surface of the device, and optionally also on at least part of the exterior surface of the device for contact with the user's fingers. An anti-bacterial material can be applied to an absorbent material (where an absorbent material is included in the device), or to the interior surface of the device, as a surface coating or treatment or can be compounded into the material (e.g. fibres/polymer/pores of an absorbent material). Preferably, commercially available anti-bacterial compositions known and appropriate for use and contact with humans and that are effective against bacterial organisms (e.g. Escherichia coli, Pseudomonas aeruginosa) are utilized. An example of such an anti-bacterial composition is Surfacine^{®} a silver-based anti-bacterial from Surfacine Development Company, Tewksbury, Massachusetts. Alternatively, an antiseptic agent agent may be used. Any suitable antiseptic agent may be applied, such as D DON-2 ^{™} available from Cosmic Discovery SDN BHD of Selangor Darul Ehsan, Malaysia, which agent is a mixture of water, silicone oil, cetylpyridinium chloride (a quaternary ammonium salt).

The term "antiseptics" is intended to mean any of a category of antimicrobial substances that inhibits the action of microorganisms, of which examples include (not limited to) chlorhexidine, methylisothiazolone, thymol, .alpha.-terpineol, cetylpyridinium chloride and chloroxylenol. These examples of antiseptics may be used by applying or impregnating an effective amount of one or a combination thereof to the at least part of the proximal portion of the device of the invention.

Preferably, where the device comprises an absorbent function and an anti-bacterial/antiseptic or anti-fungal function on at least a part of the proximal portion, said functions are contiguous. Optionally, the anti-bacterial/antiseptic and/or anti-fungal function is provided by impregnating an anti-bacterial/antiseptic and/or anti-fungal substance or substances into an absorbent material provided.

In any case, the anti-bacterial/antiseptic and/or anti-fungal function should be provided by an agent or substance that is hypoallergenic.

Optionally, the internal portion of the device comprises a cleansing fluid, which is released or applied on contact with the user, such as an encapsulated alcohol formulation.

In a particularly useful embodiment, there is provided an absorbent and cleansing portion on at least a part of the interior proximal portion and on at least a part of the exterior surface of the device, which absorbent and cleansing portions are arranged such that on stacking the absorbent cleansing portion on the internal part of one device contacts with the absorbent cleansing portion on the external portion of another. This has the advantage that where a cleansing fluid or cleansing agent is present, drying or degradation of the material is reduced due to sealing of the area during storage.

A device according to the invention or insert may be prepared, for example, by providing in sheet form a material with which to form the device, impregnating or coating at least a portion of said material with an anti-bacterial/antiseptic or anti-fungal agent, cutting said sheet material and assembling therefrom the device described above. Preferably, the manufacture is carried out in a continuous process in which the sheet material (e.g. paper) is coated, for example on a roll-to-roll process, with an absorbent material (e.g. by coating with a glue according to a specific pattern and then coating with fibres of the absorbent material) and/or an anti-bacterial/antiseptic or anti-fungal agent. Cutting and carried out in a continuous process in which the sheet material (e.g. paper) is coated, for example on a roll-to-roll process, optionally with an absorbent material (e.g. by coating with a glue according to a specific pattern and then coating with fibres of the absorbent material) and with an anti-bacterial/antiseptic or anti-fungal agent. Cutting and finishing of the sheet material to form the device of the invention can then be carried out. Preferably, in an embodiment where the device carries and image (e.g. for advertising), the manufacturing method further comprises prior to cutting and finishing of the sheet material, coating onto the sheet material a formulation for forming an image receiving (preferably ink-receiving and more preferably porous) medium, drying the coating and forming thereon (typically also prior to cutting and finishing) one or more desired images (e.g. by high-speed ink-jet printing), typically according to an arrangement of templates on the sheet (as controlled by computer) whereby on cutting and finishing a device with an image on the exterior surface is provided.

A stacking assembly for the devices of the invention is provided for dispensing of the devices, for example at a domestic or public convenience, in which the devices (or inserts) are stacked one within another to enable the device to be dispensed either distal end first or proximal end first. In one preferred embodiment, the devices are stacked in the dispenser to deliver distal end first. In this embodiment, to maximize the visibility of the images, e.g. advertising, carried on the exterior surfaces of the devices, the stacking assembly is arranged to provide a stack of devices distal end upwards, which may be dispensed from the top by removal of each device from the distal end of the stacking assembly. In this embodiment, it is preferred to utilize devices on which images have been formed to be viewed at the desired orientation when the device is orientated distal end upward.

Preferably, in a stacking assembly of devices carrying advertising or other image or message, the devices are arranged such that more than one advertising message is provided in a stacking assembly, preferably such that a consecutive device in the stacking assembly carries a different advertisement than the preceding device, whereby advertising exposure to a single user is as much as doubled. This may be particularly attractive to advertisers having more than one product.

Optionally, the distal end of the urinary directional device is crimped, such that on application of pressure of fluid during urination the distal aperture opens sufficiently to enable passage of urine through the aperture, whilst enables easier dispensing of the device from a stack thereof.

The device according to the present invention also finds application according to a further embodiment in which it is adapted for use in collecting urination samples (e.g. for medical use). The insert in particular may find application in devices intended for assisting patients in passive urination, whereby the insert allows cleanliness to be maintained during removal of the passive urination device.

The invention will now be described, without limitation as to the scope of the invention, with reference to the attached figures.

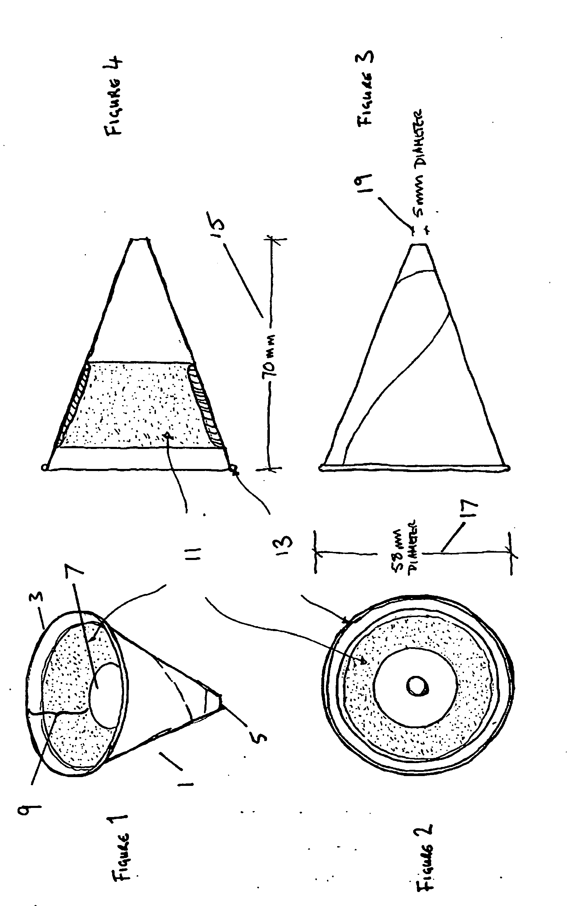

With reference to Figure 1, a directional urinary device consists of a truncated conical (or frusto-conical) funnel 1 having a conduit therethrough defined by the interior of the funnel 1 and a larger diameter proximal aperture 3 adapted to fit about the tip of the penis of a user and a smaller diameter distal aperture 5 for the passage of urine out of the conduit. The device is defined as having a distal portion 7, through which urine will pass to the distal aperture 5, and a proximal portion 9, the border between the distal portion 7 and the proximal portion 9 being about the area where the tip of the penis engages with the device in use. Typically, the surface of the proximal portion 9 will not come into substantial contact with urine during urination although leakage and spillage during or immediately after urination may cause wetting of the proximal portion 9. The proximal portion is characterized by having coating 11 (hatched area), having anti-septic or anti-fungal properties. The distal edge of the coating 11 may define or extend beyond the border of the distal portion 7 and the proximal portion 9 of the device, or it may be located and specific areas within the proximal portion 9. In the Figures 1, 2 and 4, the coating 11 is shown as covering the internal circumference of the device and, as can be seen in Figure 4, may be several millimeters thick, which depending upon the material from which it is made provides extra absorbency and also comfort and improved engagement for the wearer. In Figure 4, it can be seen that the proximal aperture 3 is defined by a rolled edge 13, which provides structural integrity to the device and assists in maintaining the shape of the proximal aperture 3 during stacking and in use as well as improve the strength of the device and prevent it from collapsing during use. The device may be manufactured to the required size, but according to the Figures, has a length 15 of about 70 mm, a proximal diameter 17 of about 58 mm and a distal diameter 19 of about 5 mm.

The device 1 may be manufactured by coating onto a web of the material from which the device 1 is made a coating 11 in a pre-determined shape, cutting the material such that it can be assembled into a truncated conical shape and sealed along edges 21 such that the coating 11 is formed in the desired configuration.

In use, the device 1 is engaged with the tip of the user's penis such that the urethral passage terminates in an area bordering or above the distal portion 7. During urination, the device 1 is directed as required to enable urine to pass through the distal aperture 5 to the target, e.g. urinal or toilet bowl. Once urination is complete, the device 1 is removed whilst allowing the tip of the penis to brush across the coating 11 which may draw up residual urine on the tip of the penis and optionally applies an anti-bacterial to the penis to improve hygiene. By locating the absorbent and/or anti-septic or anti-fungal coating in the proximal zone, preferably for positioning at the underside of the penis, improved absorbency of residual urine (preventing later spillage, dripping and soiling of hands or clothing) and optionally application of an anti-bacterial substance (to improve hygiene of the user) can be achieved simply through the action of removal of the device 1. The device 1, can then be disposed of in an hygienic matter in a conveniently provided refuse container.

The invention has been described with reference to a preferred embodiment. However, it will be appreciated that variations and modifications can be effected by a person of ordinary skill in the art without departing from the scope of the invention.

## Claims

1. A urinary directional device (1) for improving the directional urination of a male user, said device comprising a conduit for the passage of urine and having a proximal end adapted for engagement with the penis of the user and having a proximal aperture (3) to enable the passage of urine into the conduit and a distal end having a distal aperture (5) to enable the passage of urine from the conduit, said device (1) having an interior surface and an exterior surface, the device (1) being **characterized in that** the interior surface provides one or more of an anti-bacterial or antiseptic or an anti-fungal function whereby the penis of the user can be cleansed by wiping on the interior surface of the device (1) after urination.

2. A urinary directional device (1) as claimed in claim 1, said device (1) having a distal portion (7) **characterized by** providing for the passage of a stream of urine and a proximal portion (9), which during urination does not come into substantial contact with the stream of urine, wherein the anti-bacterial or antiseptic or the anti-fungal function is provided by at least part of the proximal portion (9) of the device (1) whereby the penis of the user can be cleansed by wiping with the at least part of the proximal portion (9) after urination by removal of the device.

3. A urinary directional device (1) as claimed in claim 1 or claim 2, which is frusto-conical in shape.

4. A urinary directional device (1) as claimed in any one claims 1 to 3, wherein the one or more of an anti-bacterial/antiseptic or anti-fungal function is provided by an anti-bacterial/antiseptic or anti-fungal substance or powder impregnated into the interior surface of the device (1).

5. A urinary directional device (1) as claimed in any one of the preceding claims, which device is formed of a stiffened paper, which is provided with a water-proofed or water resistant surface.

6. A urinary directional device (1) as claimed in any one of the preceding claims which has dimensions such that the length is in the range of from 60 to 80 mm, the proximal aperture (3) has a largest diameter in the range of from 50 to 60 mm and the distal aperture (5) has a largest diameter in the range of from 15 to 25 mm.

7. A urinary directional device (1) as claimed in any one of the preceding claims, wherein the circumference defining the proximal aperture is provided with a rolled edge (13).

8. A urinary directional device (1) as claimed in any one of the preceding claims, wherein at least a part of the exterior surface of the device (1) provides an absorbent and/or anti-bacterial function whereby the hand or fingers of the user can be cleansed by the action of applying and removing the device.

9. A urinary directional device (1) as claimed in any one of the preceding claims, wherein the exterior surface of the device (1) is formed of a material capable of performing as a image receiving medium for the purpose of carrying advertising, images or personalized messages.

10. A urinary directional device (1) as claimed in claim 9, wherein the exterior surface of the device (1) is a porous media which carries a water-fast ink-jet image and is capable of absorbing aqueous fluid on contact.

11. A urinary directional device (1) as claimed in any one of the preceding claims wherein the distal end is crimped, such that on application of pressure of fluid during urination the distal aperture opens sufficiently to enable passage of urine through the aperture (5), whilst enables easier dispensing of the device (1) from a stack thereof.

12. A device (1) as claimed in any one of the preceding claims, which provides an anti-bacterial function.

13. A device (1) as claimed in any one of the preceding claims, which further provides a cleansing function by having on the internal surface of the proximal portion (9) an absorbency function.

14. A device (1) as claimed in any one of the preceding claims, which further provides a cleansing function by having provided on the internal surface of the proximal portion (9) an encapsulated alcohol formulation which is released or applied on contact with the user.

15. A method of manufacturing a urinary direction device (1) as claimed in any one of the preceding claims, said method comprising providing in sheet form a material with which to form the device, impregnating or coating at least a portion of said material with an anti-bacterial or antiseptic or anti-fungal agent, cutting said sheet material and assembling therefrom the device.

16. A method as claimed in claim 15, wherein prior to cutting said sheet material, the sheet is coated on the side that will form the exterior surface of the device with a formulation to form an ink-receiving surface and printing on the ink-receiving surface one or more desired images.

17. A stacking assembly of the device (1) of any one of claims 1 to 14, said assembly comprising a dispensing component for dispensing said devices (1) and a plurality of said stacked devices (1) or inserts as defined in one of claims 1 to 14.

18. A stacking assembly as claimed in claim 17, wherein the device (1) is a substantially frusto-conical structure and the assembly is arranged such that the distal end of each device (1) is upward.

## Patentansprüche

1. Harnzielvorrichtung (1) zur Verbesserung der Harnrichtung eines männlichen Benutzers, wobei die besagte Vorrichtung eine Leitung für den Durchfluss von Harn aufweist, mit einem nahen Ende, geeignet für das Einführen des Penis des Benutzers, mit einer nahen Öffnung (3), um den Durchfluss von Harn durch die Leitung zu ermöglichen, und einem entfernten Ende mit einer entfernten Öffnung (5), um den Durchfluss von Harn durch die Leitung zu ermöglichen, die besagte Vorrichtung (1) eine Innenfläche und eine Außenfläche aufweist und die Vorrichtung (1) **dadurch gekennzeichnet ist, dass** die Innenfläche eine oder mehrere antibakterielle oder antiseptische oder antimykotische Funktionen bietet und der Penis des Benutzers nach dem Harnen durch Wischen auf der Innenfläche der Vorrichtung (1) gereinigt werden kann.

2. Harnzielvorrichtung (1) nach Anspruch 1, **dadurch gekennzeichnet, dass** die besagte Vorrichtung (1) mit einem entfernten Teil (7), um den Durchfluss eines Harnstrahls zu ermöglichen, und einem nahen Teil (9), das beim Harnen nicht grundsätzlich in Kontakt mit dem Harnstrahl kommt, versehen ist, wobei die antibakterielle oder antiseptische oder die antimykotische Funktion durch zumindest einen Teil des nahen Teils (9) der Vorrichtung (1) gewährt wird und der Penis des Benutzers nach dem Harnen durch Wischen mit zumindest dem Teil des nahen Teils (9) durch Entfernen der Vorrichtung gereinigt werden kann.

3. Harnzielvorrichtung (1) nach Anspruch 1 oder Anspruch 2 mit einer kegelstumpfartigen Form.

4. Harnzielvorrichtung (1) nach einem beliebigen der Ansprüche 1 bis 3, wobei eine oder mehrere der antibakteriellen/antiseptischen oder antimykotischen Funktionen durch eine antibakterielle/antiseptische oder antimykotische Substanz oder ein solches Pulver, auf die Innenfläche der Vorrichtung (1) imprägniert, gewährt werden.

5. Harnzielvorrichtung (1) nach einem beliebigen der vorhergehenden Ansprüche, wobei die Vorrichtung aus einem versteiften Papier gebildet wird, das mit einer wasserdichten oder wasserbeständigen Fläche versehen ist.

6. Harnzielvorrichtung (1) nach einem beliebigen der vorhergehenden Ansprüche, die so dimensioniert ist, dass die Länge im Bereich von 60 bis 80 mm liegt, die nahe Öffnung (3) einen größten Durchmesser im Bereich von 50 bis 60 mm hat und die entfernte Öffnung (5) einen größten Durchmesser im Bereich von 15 bis 25 mm hat.

7. Harnzielvorrichtung (1) nach einem beliebigen der vorhergehenden Ansprüche, wobei der die nahe Öffnung definierende Umfang mit einem gewalzten Rand (13) versehen ist.

8. Harnzielvorrichtung (1) nach einem beliebigen der vorhergehenden Ansprüche, wobei mindestens ein Teil der Außenfläche der Vorrichtung (1) eine absorbierende und/oder antibakterielle Funktion bietet, wodurch die Hand oder Finger des Benutzers durch das Benutzen und Entfernen der Vorrichtung gereinigt werden können.

9. Harnzielvorrichtung (1) nach einem beliebigen der vorhergehenden Ansprüche, wobei die Außenfläche der Vorrichtung (1) aus einem Material gebildet wird, das für das Aufbringen von Bildmaterial für Werbezwecke, Bildern oder persönliche Nachrichten geeignet ist.

10. Harnzielvorrichtung (1) nach Anspruch 9, wobei die Außenfläche der Vorrichtung (1) aus porösem Material besteht, das ein wasserfestes Tintenstrahl-Druckbild trägt und das beim Kontakt wässrige Flüssigkeit absorbieren kann.

11. Harnzielvorrichtung (1) nach einem beliebigen der vorhergehenden Ansprüche, wobei das entfernte Ende gequetscht ist, damit sich durch den beim Harnen durch die Flüssigkeit entstehenden Druck die entfernte Öffnung ausreichend öffnet, um den Durchfluss von Harn durch die Öffnung (5) zu ermöglichen, was ein leichteres Austeilen der Vorrichtung (1) aus einem Stapel heraus ermöglicht.

12. Vorrichtung (1) nach einem beliebigen der vorhergehenden Ansprüche, die eine antibakterielle Funktion bietet.

13. Vorrichtung (1) nach einem beliebigen der vorhergehenden Ansprüche, die ferner eine reinigende Funktion bietet, da sie auf der Innenfläche des nahen Teils (9) eine absorbierende Funktion hat.

14. Vorrichtung (1) nach einem beliebigen der vorhergehenden Ansprüche, die ferner eine reinigende Funktion bietet, da sie auf der Innenfläche des nahen Teils (9) eine gekapselte Alkoholformulierung aufweist, die beim Kontakt mit dem Benutzer freigegeben oder aufgebracht wird.

15. Verfahren zur Herstellung einer Harnzielvorrichtung (1) nach einem beliebigen der vorhergehenden Ansprüche, wobei das besagte Verfahren die Bereitstellung eines Materials in Blattform umfasst, mit dem die Vorrichtung geformt wird, mindestens ein Teil des besagten Materials mit einem antibakteriellen oder antiseptischen oder antimykotischen Mittel imprägniert oder beschichtet wird und das besagte Blattmaterial zugeschnitten wird und daraus die Vorrichtung gebildet wird.

16. Verfahren nach Anspruch 15, wobei vor dem Schneiden des besagten Blattmaterials die Blätter auf der Seite zum Bilden der Außenfläche der Vorrichtung mit einer Formulierung beschichtet werden, um eine Tinte aufnehmende Oberfläche zu bilden, und auf die Tinte aufnehmende Oberfläche eines oder mehrere Bilder nach Wunsch zu drucken.

17. Stapelanordnung der Vorrichtung (1) nach einem beliebigen der Ansprüche 1 bis 14, wobei die besagte Anordnung eine Austeilkomponente für das Austeilen der besagten Vorrichtungen (1) und eine Vielzahl der besagten gestapelten Vorrichtungen (1) oder Einsätze aufweist, wie in einem der Ansprüche 1 bis 14 definiert.

18. Stapelanordnung nach Anspruch 17, wobei die Vorrichtung (1) eine grundsätzlich kegelstumpfartige Struktur aufweist und die Anordnung derart angeordnet ist, dass sich das entfernte Ende jeder Vorrichtung (1) oben befindet.

## Revendications

1. Dispositif urinaire directionnel (1) pour améliorer la miction directionnelle d'un utilisateur masculin, ledit dispositif comprenant un conduit pour le passage de l'urine et ayant une extrémité proximale qui est à même de s'engager sur le pénis de l'utilisateur ainsi qu'une ouverture proximale (3) pour permettre le passage de l'urine dans le conduit et une extrémité distale ayant une ouverture distale (5) pour permettre le passage de l'urine hors du conduit, ledit dispositif (1) ayant une surface interne et une surface externe, le dispositif (1) étant **caractérisé en ce que** la surface interne assure une ou plusieurs fonctions parmi une fonction antibactérienne ou antiseptique ou encore une action antifongique, de sorte que le pénis de l'utilisateur puisse être nettoyé par essuyage sur la surface interne du dispositif (1) après miction.

2. Dispositif urinaire directionnel (1) selon la revendication 1, ledit dispositif (1) ayant une portion distale (7), **caractérisée en ce qu'**elle permet le passage d'un flux d'urine et une portion proximale (9), qui, au cours de la miction, ne vient pas en contact sensible avec le flux d'urine, dans lequel la fonction antibactérienne ou antiseptique ou la fonction antifongique est assurée par au moins une partie de la portion proximale (9) du dispositif (1), de sorte que le pénis de l'utilisateur puisse être nettoyé par essuyage avec la au moins une partie de la portion proximale (9) après miction par retrait du dispositif.

3. Dispositif urinaire directionnel (1) selon la revendication 1 ou la revendication 2, qui a une forme tronconique.

4. Dispositif urinaire directionnel (1) selon l'une quelconque des revendications 1 à 3, dans lequel la une ou plusieurs fonctions parmi une fonction antibactérienne/antiseptique ou une fonction antifongique est ou sont assurée(s) par une substance ou une poudre antibactérienne/antiseptique ou antifongique dont est imprégnée la surface interne du dispositif (1).

5. Dispositif urinaire directionnel (1) selon l'une quelconque des revendications précédentes, lequel dispositif est formé d'un papier renforcé, qui est pourvu d'une surface étanche ou résistante à l'eau.

6. Dispositif urinaire directionnel (1) selon l'une quelconque des revendications précédentes, qui a des dimensions telles que la longueur se situe dans la plage de 60 à 80 mm, que l'ouverture proximale (3) ait le plus grand diamètre dans la plage de 50 à 60 mm et que l'ouverture distale (5) ait le plus grand diamètre dans la plage de 15 à 25 mm.

7. Dispositif urinaire directionnel (1) selon l'une quelconque des revendications précédentes, dans lequel la circonférence définissant l'ouverture proximale est pourvue d'un bord arrondi (13).

8. Dispositif urinaire directionnel (1) selon l'une quelconque des revendications précédentes, dans lequel au moins une partie de la surface externe du dispositif (1) assure une fonction absorbante et/ou antibactérienne, de sorte que la main ou les doigts de l'utilisateur puisse(nt) être nettoyés par l'action d'application et de retrait du dispositif.

9. Dispositif urinaire directionnel (1) selon l'une quelconque des revendications précédentes, dans lequel la surface externe du dispositif (1) est formée d'un matériau capable de jouer le rôle d'un support récepteur d'image dans le but de porter de la publicité, des images ou des messages personnalisés.

10. Dispositif urinaire directionnel (1) selon la revendication 9, dans lequel la surface externe du dispositif (1) est un support poreux qui porte une image à jet d'encre résistant à l'eau et est à même d'absorber un fluide aqueux par contact.

11. Dispositif urinaire directionnel (1) selon l'une quelconque des revendications précédentes, dans lequel l'extrémité distale est sertie de sorte que, par application de la pression du fluide au cours de la miction, l'ouverture distale s'ouvre suffisamment pour permettre le passage de l'urine à travers l'ouverture (5), tout en permettant une distribution plus aisée du dispositif (1) à partir de sa pile.

12. Dispositif (1) selon l'une quelconque des revendications précédentes, qui assure une fonction antibactérienne.

13. Dispositif (1) selon l'une quelconque des revendications précédentes, qui assure par ailleurs une fonction de nettoyage en présentant sur la surface interne de la portion proximale (9) une fonction d'absorption.

14. Dispositif (1) selon l'une quelconque des revendications précédentes, qui assure par ailleurs une fonction de nettoyage en prévoyant sur la surface interne de la portion proximale (9) une formulation d'alcool encapsulée qui est libérée ou appliquée lors d'un contact avec l'utilisateur.

15. Procédé de fabrication d'un dispositif urinaire directionnel (1) selon l'une quelconque des revendications précédentes, ledit procédé comprenant la mise en oeuvre sous forme de feuille d'un matériau avec lequel le dispositif est formé, l'imprégnation ou le revêtement d'au moins une portion dudit matériau avec un agent antibactérien, antiseptique ou antifongique, la découpe dudit matériau en feuille et l'assemblage du dispositif à partir de ce dernier.

16. Procédé selon la revendication 15, dans lequel, avant la découpe dudit matériau en feuille, la feuille est revêtue sur la face qui formera la surface externe du dispositif d'une formulation permettant de former une surface réceptrice d'encre et d'imprimer une ou plusieurs images souhaitées sur la surface réceptrice d'encre.

17. Ensemble d'empilage du dispositif (1) selon l'une quelconque des revendications 1 à 14, ledit ensemble comprenant un composant distributeur pour distribuer lesdits dispositifs (1) et une pluralité desdits dispositifs empilés (1) ou des inserts comme défini dans l'une quelconque des revendications 1 à 14.

18. Ensemble d'empilage selon la revendication 17, dans lequel le dispositif (1) est une structure sensiblement tronconique et l'ensemble est aménagé de sorte que l'extrémité distale de chaque dispositif (1) soit tournée vers le haut.
